# EUROPÄISCHE PATENTSCHRIFT

(11) **EP 3 030 187 B1**
(45) Veröffentlichungstag und Bekanntmachung des Hinweises auf die Patenterteilung: **22.11.2017**
(21) Anmeldenummer: 14759109.3
(22) Anmeldetag: 05.08.2014
(51) Int. Cl.: A61B 90/30

(54) **OP-LEUCHTE MIT STEUERUNG**
SURGICAL LAMP HAVING CONTROL
LAMPE DE BLOC OPÉRATOIRE À COMMANDE SANS CONTACT

(30) Priorität: 05.08.2013 DE 102013215337
(43) Veröffentlichungstag der Anmeldung: 15.06.2016
(73) Patentinhaber: TRILUX Medical GmbH & Co. KG, 59759 Arnsberg (DE)
(72) Erfinder: HERMANN, Hausschulte, 59757 Arnsberg (DE)
(74) Vertreter: Lippert Stachow Patentanwälte Rechtsanwälte
(86) Internationale Anmeldenummer: PCT/EP2014/066819
(87) Internationale Veröffentlichungsnummer: WO 2015/018830

(56) Entgegenhaltungen:
- EP-A1- 2 283 790
- WO-A1-2010/146446
- GB-A- 2 423 378
- US-A1- 2012 075 832

## Beschreibung

Die Erfindung betrifft eine berührungslos steuerbare OP-Leuchte gemäß dem Oberbegriff von Anspruch 1.

Herkömmliche OP-Leuchten (Operationsleuchten) werden zum Ausleuchten eines Operationsfeldes eingesetzt, in dem an einem lebenden Körper operiert wird. Da Operationen in der Regel an einem offenen Körper erfolgen, hat die Sterilität im Operationssaal höchste Bedeutung. Insbesondere muss bei einer Operation vermieden werden, dass die Hände des Operateurs und die bei der Operation eingesetzten Werkzeuge verunreinigt werden, wie beispielsweise mit Viren oder Bakterien kontaminiert werden. Deshalb wird während einer Operation strengstens darauf geachtet, dass der Operateur, der die Operation vornimmt, nicht in Kontakt mit nichtsterilisierten Gegenständen kommt.

Entsprechend wird während einer Operation auch streng vermieden, dass der Operateur in Kontakt mit der OP-Leuchte kommt, die als solche, bis auf einen einzelnen Fokussiergriff, nicht sterilisierbar ist. Dabei stellt sich das Problem, dass für die Operation eine möglichst optimale Ausleuchtung des Operationsfeldes durch die Operationsleuchte notwendig ist, wobei hierfür die Ausleuchtung nach den Bedürfnissen des behandelnden Operateurs im Hinblick auf die zu operierende Wunde auszurichten ist. Beispielsweise ist es vorteilhaft, wenn die Lichtfarbe, die Lichtintensität, die Richtung, mit der Licht von der OP-Leuchte auf das Operationsfeld gesendet wird, und/oder die Größe des ausgeleuchteten Bereichs, d. h. der Fokus der OP-Leuchte, nach den Bedürfnissen des Operateurs einstellbar ist. Diese Ausleuchtungseigenschaften der OP-Leuchte lassen sich üblicherweise über Parameter der OP-Leuchte einstellen, die dann den Ausleuchtungsbereich im Operationsfeld den Einstellungen entsprechend ausleuchtet. Da der Operateur die nicht-sterile Operationsleuchte bis auf den Fokus selbst nicht bedienen kann und somit nicht so steuern kann, dass die OP-Leuchte die von ihm gewünschte Abstrahl- bzw. Ausleuchtungscharakteristik aufweist, muss die Steuerung der OP-Leuchte durch Personen vorgenommen werden, die nicht unmittelbar an der Operation beteiligt sind und damit nicht zur Verunreinigung von offenen Stellen am Körper wegen einer Kontamination über die OP-Leuchte beitragen können. In der Operationspraxis muss daher der Operateur diese Personen zur Steuerung der OP-Leuchte anweisen und kann die Steuerung der OP-Leuchte nicht selbst vornehmen. Dies ist in der Praxis insbesondere deswegen problematisch, weil während einer Operation verschiedene Operationssituationen auftreten, die jeweils eine andere Ausleuchtung des Operationsfeldes erfordern. Der Operateur muss stets den die OP-Leuchte bedienenden Personen seine sich ändernden Bedürfnisse in Bezug auf die Ausleuchtungscharakteristik der OP-Leuchte erläutern, die dann die Steuerung der OP-Leuchte vornehmen, was Zeitverzögerungen bei der Operation und eine oftmals nicht optimale Ausleuchtung des Operationsfeldes mit sich bringt.

Im Stand der Technik wird den genannten Problemen dadurch Rechnung getragen, dass an einer OP-Leuchte eine Steuereinheit und eine mit der Steuereinheit korrespondierende Sensoreinheit angeordnet wird. Die Sensoreinheit ist mit einer sterilen Abdeckung bedeckt, die die Funktionsfähigkeit der Sensoreinheit nicht beeinträchtigt. Somit kann mit einem sterilen Aktionselement an der sterilen Abdeckung der Sensoreinheit entlanggefahren werden, wodurch die Sensoreinheit Bewegungsdaten generiert. Als Aktionselement kann beispielsweise eine Hand oder ein Werkzeug, das bei der Operation eingesetzt wird, verwendet werden. Die Steuereinheit liest die von der Sensoreinheit generierten Bewegungsdaten aus und steuert die OP-Leuchte in Abhängigkeit von den ausgelesenen Bewegungsdaten. Je nachdem, welche Bewegung das Aktionselement an der Abdeckung der Sensoreinheit ausgeführt hat, steuert die Steuereinheit die OP-Leuchte. Das Steuern der OP-Leuchte kann dabei beispielsweise das Einstellen von Parametern der OP-Leuchte umfassen, wodurch eine Abstrahlcharakteristik der OP-Leuchte und damit eine Ausleuchtungscharakteristik der OP-Leuchte in dem Operationsfeld eingestellt wird. Beispielsweise können die oben beschriebenen Ausleuchtungseigenschaften der OP-Leuchte eingestellt werden. Beispielsweise können durch die entsprechende Steuerung über eine entsprechende Einstellung der Parameter der OP-Leuchte die oben beschriebenen Ausleuchtungseigenschaften der OP-Leuchte vorgegeben werden. Beispielsweise kann durch die Steuerung der Fokus, insbesondere die Fokusgröße, der OP-Leuchte, und damit die Größe der Fläche des ausgeleuchteten Bereichs in dem Operationsfeld, die Lichtfarbe des von der OP-Leuchte abgestrahlten Lichts, die Intensität des abgestrahlten Lichts, der Abstand der OP-Leuchte von dem Operationsfeld und/oder die Abstrahlrichtung, mit der die OP-Leuchte Licht auf das Operationsfeld abstrahlt, einstellbar sein. Eine entsprechende vorteilhafte OP-Leuchte ist aus dem Dokument EP 2 4342 02 A1 bekannt. Eine weitere OP-Leuchte ist beispielsweise aus dem Dokument EP 2 283 790 A1 bekannt, bei der eine Steuerung der OP-Leuchte dazu ausgebildet ist, einen Handbereich von medizinischem Personal zu erkennen und Bewegungen des Handbereichs aufzunehmen und in ein Bildsignal umzuwandeln, das einem bestimmten Steuersignal zugeordnet ist, mit dem die Steuerung die OP-Leuchte steuert. Ferner ist in dem Dokument WO 2010/146446 A1 eine gattungsfremde Leuchte offenbart, bei der eine berührungslose Steuerung der Leuchte dadurch erfolgt, dass Bewegungen einer Hand erfasst werden und in ein Steuersignal umgewandelt werden, wobei diese berührungslose Steuerung nur dann erfolgt, wenn die Steuerung sich in einem Bereitschaftsmodus befindet, wobei der Bereitschaftsmodus durch eine vorbestimmte Handbewegung ein- und ausgeschaltet wird.

Die bekannten OP-Leuchten gewährleisten somit eine berührungslose Steuerung der OP-Leuchte in dem Sinne, dass nur eine sterile Abdeckung zur Steuerung der OP-Leuchte berührt werden muss, so dass eine Steuerung der OP-Leuchte durch den Operateur selbst durchführbar ist. Die bekannten OP-Leuchten weisen jedoch verschiedene Nachteile auf. Beispielsweise muss der Operateur zur Steuerung der OP-Leuchte seine Hand zur Sensoreinheit hinbewegen und damit weit von dem Operationsfeld entfernen, was der Operateur als störend empfindet. Beispielsweise wird der Operateur von der Operation erheblich abgelenkt, wenn er die sterile Abdeckung gezielt zu erreichen versucht. Beispielsweise ist wegen der geringen Größe des Sensorelements, mit dem die Sensoreinheit Bewegungen aufnehmen kann, eine Steuerung der OP-Leuchte nur in geringem Umfang durch den Operateur möglich. Damit ist der Operateur weiterhin auf eine zusätzliche Steuerung der OP-Leuchte durch andere Personen angewiesen. Beispielsweise kann die Sensoreinheit wegen der erforderlichen sterilen Abdeckung fehleranfällig sein, da die sterile Abdeckung bei einer fehlerhaften Montage das Generieren von Bewegungsdaten durch die Sensoreinheit erschweren oder unmöglich machen oder verfälschen kann.

Der Erfindung liegt die Aufgabe zugrunde, eine OP-Leuchte bereitzustellen, die berührungslos steuerbar ist, insbesondere durch den Operateur selbst, und die die obengenannten Probleme von bekannten OP-Leuchten zumindest teilweise behebt.

Als eine Lösung der beschriebenen technischen Aufgabe schlägt die Erfindung eine OP-Leuchte mit den Merkmalen von Anspruch 1 vor. Diese erfindungsgemäße OP-Leuchte zeichnet sich dadurch aus, dass als Deaktivierungsaktion festgelegt ist, dass während einer vorbestimmbaren Totzeit keine Bewegung des Aktionselements erfasst wurde. Die erfindungsgemäße OP-Leuchte bringt mehrere Vorteile mit sich. Dadurch, dass die Sensoreinheit so ausgebildet ist, dass sie die Erfassung der Bewegung des Aktionselements in drei Dimensionen gewährleistet, kann die Sensoreinheit zu einer sehr großen Anzahl an verschiedenen Bewegungen, die dreidimensional ausgeführt werden können, jeweils verschiedene Bewegungsdaten generieren. Diese verschiedenen Bewegungsdaten kann die Steuereinheit jeweils auslesen, so dass eine umfassende Steuerung der OP-Leuchte und damit eine Einstellung von insbesondere sämtlichen relevanten Eigenschaften der Abstrahlcharakteristik der OP-Leuchte durch entsprechende Bewegungen des Aktionselements möglich sein kann. In einer Ausführungsform kann die Anzahl der Steuerungsmöglichkeiten dadurch noch vergrößert sein, dass die Sensoreinheit dazu ausgebildet ist, Bewegungen eines mehrteiligen Aktionselements zu erfassen. In einem Ausführungsbeispiel kann das Aktionselement beispielsweise aus zwei Teilen bestehen, wie etwa zwei Händen oder zwei Werkzeugen. Beispielsweise kann die Sensoreinheit dazu ausgebildet sein, die Relativbewegung der beiden Teile des Aktionselements zueinander zu erfassen und die Relativbewegung in Bewegungsdaten umzuwandeln, die dann von der Steuereinheit ausgelesen werden können. Durch das Erfassen einer Relativbewegung von zwei Teilen des Aktionselements kann eine besonders präzise Generierung von Bewegungsdaten und damit eine besonders präzise Steuerung der OP-Leuchte durch die Steuereinheit gewährleistet sein. In einer anderen Ausführungsform ist die Sensoreinheit dazu ausgebildet, den Bewegungsablauf des Aktionselements aufzunehmen in dem Sinne, dass die Sensoreinheit Bewegungsdaten generiert, die die Ortsveränderung des Aktionselements während der Bewegung wiederspiegeln. In einer weiteren Ausführungsform kann die Sensoreinheit dazu ausgebildet sein, die Geschwindigkeit und/oder die Beschleunigung des Aktionselements zu erfassen und bei der Generation der Bewegungsdaten zu berücksichtigen. Die Merkmale der genannten Ausführungsformen können auch miteinander kombiniert werden.

Der Fachmann kann auf bekannte Ausgestaltungen von Sensoreinheiten zurückgreifen zur Realisierung einer Sensoreinheit, die das Erfassen von dreidimensionalen Bewegungen in dem Messraum ermöglicht. Beispielsweise kann die Sensoreinheit aus einem Leap-Sensor bestehen oder aus einer 3D-Kamera. Beispielsweise kann die Sensoreinheit aus mehreren Kameras bestehen, die ortsversetzt zueinander angeordnet sind und damit eine dreidimensionale Erfassung von Bewegungen in dem Messraum ermöglichen. Beispielsweise kann die Sensoreinheit aus mehreren Leap-Sensoren bestehen. Beispielsweise kann die Sensoreinheit aus mehreren Infrarotsensoren bestehen, die so ortsversetzt zueinander angeordnet sind, dass eine dreidimensionale Erfassung von Bewegungen eines Aktionselements mit einer bestimmten Temperatur in dem Messraum möglich ist. Beispielsweise kann die Sensoreinheit zumindest eine, insbesondere mehrere, insbesondere verschiedene der beschriebenen oder anderer Sensorelemente, wie etwa Leap-Sensor, 3D-Kamera, Infrarotsensor, umfassen.

Die erfindungsgemäße OP-Leuchte zeichnet sich wie erläutert dadurch aus, dass über die Sensoreinheit die Bewegungen in einem Messraum erfasst werden können, der in Abstrahlrichtung unterhalb der OP-Leuchte angeordnet ist. Der Messraum erstreckt sich somit zwischen der OP-Leuchte und dem Operationsfeld. Daher muss der Operateur zur Steuerung der OP-Leuchte nicht seine Hand weit von dem Operationsfeld entfernen. In einem Ausführungsbeispiel liegt das Operationsfeld selbst in dem Messraum, in einem anderen Ausführungsbeispiel liegt das Operationsfeld in Abstrahlrichtung von dem Messraum beabstandet unterhalb des Messraums. Der Operateur muss jeweils seine Hand zur Durchführung der Bewegung zur Steuerung der OP-Leuchte nur in den Messraum hineinbewegen, der wie erläutert zwischen OP-Leuchte und Operationsfeld und damit in der Nähe des Operationsfeldes liegt. Vorzugsweise ist der Messraum der OP-Leuchte so nah an dem Operationsfeld angeordnet, dass der Operateur seine Hand in dem Messraum bewegen kann, indem er in seiner Operationsposition verharrt und nur eine Arm-und/oder Handbewegung durchführt. Vorzugsweise ist der Messraum so groß ausgebildet, dass der Operateur die Bewegungen in dem Messraum durchführen kann, ohne sich wegen einer starken räumlichen Begrenzung erheblich darauf konzentrieren zu müssen.

Der Messraum kann beispielsweise in einer Richtung senkrecht zur optischen Achse der OP-Leuchte von der optischen Achse beabstandet sein. Die optische Achse der OP-Leuchte ist als Gerade, die in Abstrahlrichtung durch die Mitte des Bereichs, in dem die OP-Leuchte Licht abstrahlt, verläuft, definiert. Dadurch kann beispielsweise gewährleistet sein, dass die Sensoreinheit eine Bewegung des Operateurs in dem Operationsfeld, das durch die OP-Leuchte ausgeleuchtet ist, nicht erfasst, so dass nicht zufällig durch eine Bewegung des Operateurs eine Steuerung der OP-Leuchte, die ungewünscht sein kann, erfolgt. In einer vorteilhaften Ausführungsform verläuft eine optische Achse, entlang der die OP-Leuchte Licht abstrahlt, durch den Messraum. Diese Ausführungsform kann den Vorteil mit sich bringen, dass der Operateur eine Steuerung der OP-Leuchte durchführen kann, indem er seine Hand innerhalb des von der OP-Leuchte ausgeleuchteten Bereichs bewegt, so dass der Operateur sich nur sehr geringfügig aus seiner Operationsposition heraus bewegen muss. In einer vorteilhaften Ausführungsform ist die Sensoreinheit so ausgebildet, dass sie eine dreidimensionale Bewegung des Aktionselements in einem Messraum gewährleistet, der ein Volumen von 1 m³ aufweist. In anderen Ausführungsformen kann ein entsprechendes Volumen des Messraums zwischen ca. 0,3 und 1 m³ eingestellt sein. Bei der Wahl der Größe des Messraums wird darauf geachtet, eine für den Operateur angenehme Größe des Messraums bereitzustellen. In einer vorteilhaften Ausführungsform ist der Messraum in einer Ebene senkrecht zur optischen Achse symmetrisch um die optische Achse angeordnet. Unter Berücksichtigung der für die Beschreibung der Erfindung verwendeten Definition der optischen Achse, die darin besteht, dass die optische Achse in Abstrahlrichtung der OP-Leuchte verläuft und durch die Mitte des Bereichs, in den die OP-Leuchte Licht abstrahlt, ist bei der genannten vorteilhaften Ausführungsform gewährleistet, dass der Operateur davon ausgehen kann, dass seine Bewegungen von der Sensoreinheit erfasst werden, wenn er sie innerhalb des von der OP-Leuchte ausgeleuchteten Bereichs durchführt. Dadurch kann der Operateur besonders einfach die Erfassung der von ihm durchgeführten Bewegungen sicherstellen.

In einer vorteilhaften Ausführungsform weist die Sensoreinheit genau einen Sensor auf, der an einer Sensorposition an der OP-Leuchte angeordnet ist, wobei der Messraum um die Sensorposition symmetrisch ist. Insbesondere kann der Messraum eine Form nach Art eines Zylinders aufweisen, wobei die Sensorposition in dem Zentrum der Grundfläche des Zylinders liegen kann. Insbesondere kann der Messraum eine Form nach Art eines Kugelabschnitts aufweisen, wobei die Sensorposition im Zentrum der Grundfläche des Kugelabschnitts angeordnet sein kann. Der Sensor ist entsprechend zur Realisierung des Messraums ausgebildet. Die vorteilhafte Ausführungsform kann den Vorteil mit sich bringen, dass mit nur einem Sensor ein Messraum gewährleistet sein kann, der so gelegen ist und eine solche Abmessung aufweist, dass er eine besonders gute Steuerung der OP-Leuchte gewährleistet. Hierzu kann insbesondere die Sensorposition je nach Bedarf festgelegt sein. Insbesondere kann die vorteilhafte Ausführungsform eine kostengünstige Herstellung einer präzise steuerbaren OP-Leuchte ermöglichen.

Erfindungsgemäß weist die OP-Leuchte
einen Ruhemodus auf, in dem die Steuereinheit keine Steuerung der OP-Leuchte über die ausgelesenen Bewegungsdaten gewährleistet, sowie einen Bereitschaftsmodus, in dem die Steuereinheit die Steuerung der OP-Leuchte über die ausgelesenen Bewegungsdaten gewährleistet, wobei die Steuereinheit so ausgebildet ist, dass durch eine Initialisierungsaktion der Wechsel von dem Ruhemodus in den Bereitschaftsmodus und durch eine Deaktivierungsaktion der Wechsel von dem Bereitschaftsmodus in den Ruhemodus durchführbar ist. In dem Ruhemodus ist somit keine Steuerung der OP-Leuchte durch eine Bewegung möglich.

Beispielsweise kann die OP-Leuchte so ausgebildet sein, dass die Steuereinheit in dem Ruhemodus keine von der Sensoreinheit generierten Bewegungsdaten ausliest. Beispielsweise kann die OP-Leuchte so ausgebildet sein, dass in dem Ruhemodus die Sensoreinheit keine Bewegungen erfasst und somit keine Bewegungsdaten generiert. In einer Ausführungsform liest die Steuereinheit in dem Ruhemodus die Bewegungsdaten aus, führt jedoch nicht in Abhängigkeit von den ausgelesenen Bewegungsdaten eine Steuerung durch. In dem Bereitschaftsmodus kann dagegen eine Steuerung der OP-Leuchte über eine Bewegung in dem Messraum erfolgen. Entsprechend erfasst die Sensoreinheit in dem Bereitschaftsmodus der OP-Leuchte eine Bewegung des Aktionselements in dem Messraum, und die Steuereinheit steuert die OP-Leuchte in Abhängigkeit von den Bewegungsdaten, die sie von der Sensoreinheit ausgelesen hat.

Die Initialisierungsaktion kann beispielsweise in einer Betätigung der OP-Leuchte bestehen. Beispielsweise kann die Initialisierungsaktion in der Aktion bestehen, dass ein Schalter bei der OP-Leuchte betätigt wird, um die OP-Leuchte in den Bereitschaftsmodus zu bringen. Entsprechend kann die Deaktivierungsaktion in der Aktion bestehen, einen Schalter zu betätigen, um den Wechsel vom Bereitschaftsmodus in den Ruhemodus durchzuführen. Die Initialisierungsaktion und die Deaktivierungsaktion können jeweils auch mehrere Aktionen umfassen, beispielsweise eine vorbestimmte Bewegung in dem Messraum und/oder eine vorbestimmte Annäherung des Aktionselements an die Sensoreinheit und/oder eine vorbestimmte Bewegung. Beispielsweise kann die Initialisierungsaktion eine vorbestimmte Kombination von Aktionen umfassen. Erfindungsgemäß besteht die Deaktivierungsaktion darin, dass während einer bestimmten Totzeit keine Bewegung des Aktionselements in dem Messraum erfasst wurde, wodurch automatisch der Wechsel von dem Bereitschaftsmodus in den Ruhemodus erfolgt.

In einer vorteilhaften Ausführungsform ist als Initialisierungsaktion eine Initialisierungsbewegung des Aktionselements in dem Messraum festgelegt. Beispielsweise kann bei diesem Ausführungsbeispiel die OP-Leuchte so ausgebildet sein, dass ein Wechsel von dem Ruhemodus in den Bereitschaftsmodus nur durch die Durchführung einer vorbestimmten, festgelegten Bewegung in dem Messraum erreicht werden kann. Beispielsweise kann als Initialisierungsbewegung festgelegt werden, dass das Aktionselement zweimal mit einer Geschwindigkeit, die höher als eine vorbestimmte Mindestgeschwindigkeit ist, senkrecht zur optischen Achse in dem Messraum bewegt wird. Beispielsweise kann über entsprechende Vorgabe einer Mindestgeschwindigkeit als Initialisierungsbewegung festgelegt werden, dass zwei Teile eines Aktionselements, wie beispielsweise zwei Hände, schnell gegeneinander gekreuzt bewegt werden. Insbesondere ist es vorteilhaft, die Initialisierungsbewegung so festzulegen, dass die Erfassung der relativen Bewegung des Aktionselements bei der Bewegung möglich ist. Dadurch kann erreicht werden, dass die Sensoreinheit die Initialisierungsbewegung auch dann zuverlässig erkennt, wenn die Sensoreinheit nicht zur Erfassung einer absoluten Position des Aktionselements in dem Messraum geeignet ist.

In einem Ausführungsbeispiel generiert die Sensoreinheit auch in dem Ruhemodus stets Bewegungsdaten. In einem Ausführungsbeispiel generiert die Sensoreinheit auch in dem Ruhemodus Bewegungsdaten in Abhängigkeit von einer Bewegung des Aktionselements in dem Messraum, und die Steuereinheit liest die Bewegungsdaten auch in dem Ruhemodus aus und steuert lediglich nicht die OP-Leuchte in dem Ruhemodus. Bei Durchführung einer Initialisierungsbewegung wird diese von der Sensoreinheit erfasst, von der Steuereinheit ausgelesen und dadurch die Steuereinheit zum Steuern der OP-Leuchte aktiviert.

In einer vorteilhaften Ausführungsform ist die OP-Leuchte so ausgebildet, dass die Steuereinheit die OP-Leuchte nur in Abhängigkeit von solchen Bewegungsdaten steuert, die die Sensoreinheit zu den Bewegungen des Aktionselements generiert, das die Initialisierungsbewegung vollzogen hat. Dadurch kann ausgeschlossenen sein, dass eine Steuerung der OP-Leuchte über die Bewegung eines Elements in dem Messraum durchgeführt wird, das nicht das Aktionselement ist. Somit kann eine unerwünschte Steuerung der OP-Leuchte durch zufällige Bewegung eines Elements in dem Messraum vermieden werden. In einem Ausführungsbeispiel, in dem das Aktionselement zweiteilig ausgebildet ist, kann die Initialisierungsbewegung aus einer relativen Bewegung der zwei Teile des Aktionselements gegeneinander bestehen, wonach dann die Sensoreinheit nur noch Bewegungen der beiden Teile des Aktionselements erfasst und in Bewegungsdaten umwandelt, so dass eine Steuerung durch die beiden Teile des Aktionselements möglich ist. Dabei verfolgt die Sensoreinheit im Zusammenspiel mit der Steuereinheit die Bewegungen des Aktionselements nach Beendigung der Initialisierungsbewegung. Beispielsweise kann vorgesehen sein, dass nach der Initialisierungsbewegung eine bestimmte Pausenzeit des Aktionselements, beispielsweise 2 Sekunden, vorgesehen werden muss, bevor die Steuerung der OP-Leuchte über eine Bewegung des erfassten Aktionselements erfolgen kann.

Erfindungsgemäß ist als Deaktivierungsaktion festgelegt, dass während einer vorbestimmten Totzeit keine Bewegung des Aktionselements erfasst wurde. Entsprechend kann von dem Bereitschaftsmodus in den Ruhemodus auf einfache Weise dadurch gewechselt werden, dass das Aktionselement während der Totzeit in dem Messraum ruht bzw. in dem Messraum keine Bewegung durchführt. In einer anderen Ausführungsform ist als Deaktivierungsaktion eine Deaktivierungsbewegung des Aktionselements in dem Messraum festgelegt. Die Deaktivierungsbewegung kann wie oben zur Initialisierungsbewegung beschrieben festgelegt sein.

In einem Ausführungsbeispiel ist die Sensoreinheit der OP-Leuchte dazu ausgebildet, Bewegungen von nur genau einem Aktionselement zu einer Zeit zu erfassen, wobei die Sensoreinheit die Bewegung des Aktionselements bis zum Abschluss der Bewegung ununterbrochen erfasst und es nach einer vorbestimmten Ruhezeit des Aktionselements die Erfassung eines anderen Aktionselements gewährleistet. Dadurch kann sichergestellt sein, dass nicht unbeabsichtigt Bewegungsdaten durch die Sensoreinheit generiert werden, die auf die Bewegung eines Elements in dem Messraum beruhen, das nicht dem Aktionselement entspricht, mit dem eine Person, insbesondere der Operateur, die Steuerung der OP-Leuchte erreichen möchte. Darüber hinaus kann dadurch gewährleistet sein, dass nach dem Einhalten einer vorbestimmten Ruhezeit, beispielsweise von 3 Sekunden, ein Wechsel von einem Aktionselement zu einem anderen Aktionselement erfolgen kann, so dass nach einer Steuerung der OP-Leuchte mit einem ersten Aktionselement die Steuerung der OP-Leuchte mit einem zweiten Aktionselement möglich ist. Dies kann beispielsweise dann vorteilhaft sein, wenn der Operateur mit einer Hand eine erste Bewegung für eine erste Steuerung der OP-Leuchte durchgeführt hat und danach die Hand aus Operationsgründen nicht mehr bewegen kann und somit die zweite Hand für eine zweite Steuerung der OP-Leuchte verwenden kann. In einer Ausführungsform, bei der als Deaktivierungsaktion festgelegt ist, dass während einer vorbestimmten Totzeit keine Bewegung des Aktionselements erfasst wurde, kann die Ruhezeit kleiner festgelegt sein als die Totzeit, so dass der Wechsel von einem Aktionselement zu einem anderen Aktionselement möglich ist, bevor die OP-Leuchte von dem Bereitschaftsmodus in den Ruhemodus wechselt.

In einer Ausführungsform umfasst die OP-Leuchte ein Anzeigeelement, das eine Bereitschaft der OP-Leuchte bzw. der Sensoreinheit der OP-Leuchte zur Erfassung des anderen Aktionselements anzeigt. Dadurch kann der Operateur dem Anzeigeelement entnehmen, ob eine Steuerung der OP-Leuchte über das andere Aktionselement möglich ist.

Ferner kann es vorteilhaft sein, dass die OP-Leuchte eine Anzeigeeinheit umfasst, die anzeigt, ob die OP-Leuchte durch eine Bewegung des Aktionselements steuerbar ist. Beispielsweise kann die Anzeigeeinheit anzeigen, ob sich die OP-Leuchte in einem Bereitschaftsmodus befindet. Beispielsweise kann eine entsprechende Anzeige der Anzeigeeinheit dadurch erfolgen, dass die Anzeigeeinheit einen hörbaren Ton aussendet, wenn die OP-Leuchte im Bereitschaftsmodus ist. Beispielsweise kann die Anzeigeeinheit eine Anzeigeleuchte umfassen, die aufleuchtet, wenn die OP-Leuchte sich im Bereitschaftsmodus befindet. Beispielsweise kann die Anzeigeeinheit eine Anzeigeleuchte aufweisen, die mit einer ersten Farbe leuchtet, wenn sich die OP-Leuchte in dem Ruhemodus befindet, und mit einer zweiten Farbe, wenn sich die OP-Leuchte in dem Bereitschaftsmodus befindet. In einem Ausführungsbeispiel ist das Anzeigeelement in der Anzeigeeinheit integriert, so dass die Anzeigeeinheit die Funktion des Anzeigeelements mitumfasst. Die Anzeige durch das Anzeigeelement kann wie in Bezug auf die Anzeige der Anzeigeeinheit erläutert erfolgen.

Im Folgenden wird die Erfindung anhand von zwei Figuren näher erläutert.

Es zeigen:
- Figur 1:: in einer Prinzipdarstellung die Seitenansicht einer erfindungsgemäßen OP-Leuchte;
- Figur 2:: in einer Prinzipdarstellung die Ansicht einer erfindungsgemäßen OP-Leuchte von unten.

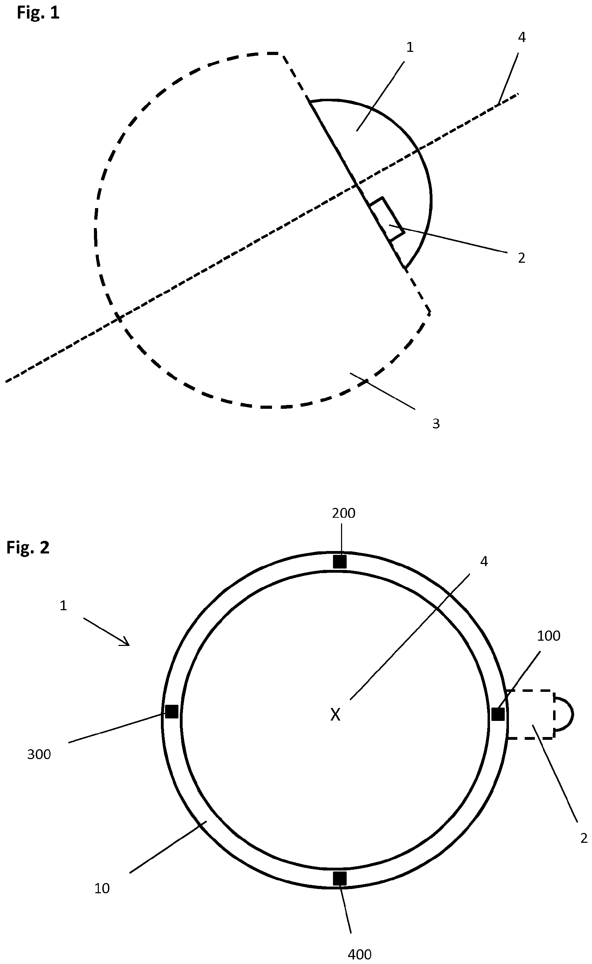

In Figur 1 ist in einer Prinzipdarstellung die Seitenansicht einer erfindungsgemäßen OP-Leuchte 1 und schematisch der in Abstrahlrichtung unterhalb der OP-Leuchte 1 angeordnete Messraum 3 dargestellt. Ferner ist in Figur 1 die optische Achse 4 eingezeichnet, die in Abstrahlrichtung der OP-Leuchte 1 verläuft und in der Mitte des von der OP-Leuchte 1 in einer Ebene, die senkrecht zur optischen Achse 4 verläuft, ausgeleuchteten Bereichs liegt.

In Figur 1 ist lediglich eine zweidimensionale Darstellung der Seitenansicht der beschriebenen Ausführungsform der erfindungsgemäßen OP-Leuchte 1 dargestellt. Der Messraum 3 weist eine Form nach Art eines Kugelabschnitts auf. Die Sensoreinheit der dargestellten erfindungsgemäßen OP-Leuchte 1 weist einen Sensor 2 auf, der am in Abstrahlrichtung unteren Rand der OP-Leuchte 1 angeordnet ist und der so ausgebildet ist, dass er Bewegungen eines Aktionselements in drei Dimensionen innerhalb des dargestellten Messraums 3 erfassen kann. In dem dargestellten Ausführungsbeispiel wird als Sensor 2 ein herkömmlicher Leap-Sensor eingesetzt. Ein herkömmlicher Leap-Sensor zeichnet sich dadurch aus, dass er dreidimensionale Bewegungen mit sehr hoher Präzision erfassen kann. In einer Ausführungsform umfasst der Leap-Sensor zwei im Infraroten aufnahmefähige Kameras und drei Infrarot-LEDs. Die Komponenten des Leap-Sensors sind so angesteuert, dass die Kameras sehr häufig ausgelesen werden können, so dass der Leap-Sensor eine Bestimmung der Position eines zu überwachenden Elements üblicherweise über 200 mal pro Sekunde durchführen kann. Darüber hinaus ermöglicht ein herkömmlicher Leap-Sensor eine präzise Positionsbestimmung von Elementen in einem Messraum von ca. 0,8 m³, der im Wesentlichen die Form eines Kugelabschnitts aufweist. Damit ist bei einer erfindungsgemäßen OP-Leuchte 1, die einen Leap Sensor als Sensor 2 umfasst, eine sehr präzise Ansteuerung der OP-Leuchte 1 gewährleistet. In anderen Ausführungsformen kann möglicherweise auch durch andere Sensortypen eine entsprechend präzise Steuerung der erfindungsgemäßen OP-Leuchte gewährleistet sein.

In der vorliegenden Ausführungsform ist der Sensor 2 der Sensoreinheit der OP-Leuchte 1 am Rahmen der OP-Leuchte 1 befestigt. Die aktiven Sensorelemente des Sensors 2 sind so ausgerichtet, dass der Sensor 2 dreidimensionale Bewegungen in dem Messraum 3, der in Abstrahlrichtung unterhalb der OP-Leuchte 1 liegt, erfassen kann und den Bewegungen entsprechende Bewegungsdaten generieren kann. In dem vorliegenden Ausführungsbeispiel weist der Messraum eine Länge von ca. 1100 mm, einen Durchmesser von ca. 1000 mm und damit ein Volumen von ca. 0,9 m³ auf. In anderen Ausführungsformen können auch andere geometrische Formen des Messraums und andere Volumina, beispielsweise Volumina von 0,6 bis 1 m³, vorgesehen sein. Die Grundfläche des Messraums 3, die senkrecht zur optischen Achse 4 verläuft, kann beispielsweise, wie in dem vorliegenden Ausführungsbeispiel, die Form eines Kreises aufweisen, beispielsweise die Form eines Quadrats oder eines sonstigen Polygons.

Die beschriebene erfindungsgemäße OP-Leuchte 1 ist so ausgebildet, dass ein Operateur die OP-Leuchte 1 mit Bewegungen steuern kann, sobald er die Bewegungen mit einem Aktionselement innerhalb des Messraums 3 durchführt, d. h. sobald er ein Aktionselement in entsprechender Nähe zur OP-Leuchte unterhalb der OP-Leuchte 1 bewegt. In dem vorliegenden Ausführungsbeispiel sind in der in Figur 1 nicht dargestellten Steuereinheit der OP-Leuchte 1 vorgegebene Parameter(Einstellparameter) für die OP-Leuchte 1 abgelegt, die entsprechenden vorgegebenen Bewegungsdaten zugeordnet sind. Die Steuereinheit gleicht die Bewegungsdaten, die sie aus der Sensoreinheit ausliest, mit den abgelegten Bewegungsdaten ab und steuert dann die OP-Leuchte 1 bei Übereinstimmung von ausgelesenen Bewegungsdaten mit abgelegten Bewegungsdaten entsprechend den Einstellparametern, die zu den entsprechenden Bewegungsdaten hinterlegt sind. Eine entsprechende Zuordnung von Bewegungsdaten und Parametern und ein entsprechender Abgleich von Bewegungsdaten kann auch in anderen Ausführungsbeispielen realisiert sein.

In der Steuereinheit der beschriebenen OP-Leuchte 1 ist hinterlegt, dass als Aktionselement nur eine Hand oder mehrere Hände akzeptiert werden. Entsprechende Vorgaben für ein zwingend erforderliches Aktionselement können in anderen Ausführungsformen auf andere Art und Weise gemacht werden. Beispielsweise kann eine bestimmte Markierung, beispielsweise über einen RFID, erforderlich sein, damit ein Aktionselement zur Steuerung der OP-Leuchte akzeptiert wird und damit aus Bewegungen dieses Aktionselements Bewegungsdaten generiert werden. Eine entsprechende, in der Steuereinheit abgelegte Bedingung für ein Aktionselement kann den Vorteil mit sich bringen, dass eine versehentliche Steuerung der OP-Leuchte 1 über sich bewegende Elemente ausgeschlossen sein kann. Beispielsweise kann auf einem Handschuh des Operateurs eine entsprechende Markierung vorgesehen sein, die von der Sensoreinheit erfasst werden muss, damit aus der Bewegung des mit der Markierung versehenen Aktionselements, im angegebenen Beispiel des Handschuhs mit der Markierung, Bewegungsdaten generiert werden. In einem Ausführungsbeispiel, das nicht dargestellt ist, ist für die Aktivierungsaktion zum Wechsel vom Ruhemodus in den Bereitschaftsmodus der OP-Leuchte 1 das Erkennen einer bestimmten Markierung durch die Sensoreinheit erforderlich.

In dem vorliegenden Ausführungsbeispiel ist unter der Voraussetzung, dass eine Hand als Aktionselement verwendet wird, folgende Zuordnung von Bewegungsdaten zu einer vorbestimmten Steuerung, insbesondere zu Einstellparametern hinterlegt: Werden Bewegungsdaten ausgelesen, die eine Bewegung der Hand entlang der optischen Achse 4 und zur OP-Leuchte 1 hin repräsentieren, wird die Intensität des von der OP-Leuchte 1 abgestrahlten Lichts verringert. Bei Bewegungsdaten, die eine entsprechende Bewegung der Hand von der OP-Leuchte 1 weg repräsentieren, wird die Beleuchtungsintensität entsprechend erhöht. Die Quantität der Steigerung bzw. Verringerung der Intensität entspricht der Wegstrecke, die die Hand entlang der optischen Achse 4 durchfährt. Weiterhin ist hinterlegt, dass der Durchmesser des von der OP-Leuchte 1 ausgeleuchteten Bereichs verkleinert wird, wenn eine Bewegung erkannt wird, die der Bewegung von Daumen und Zeigefinger der Hand entspricht, wenn sich Daumen und Zeigefinger vom beabstandeten Zustand zu einem geschlossenen Kreis nähern. Entsprechend ist hinterlegt, dass der Durchmesser des ausgeleuchteten Bereichs vergrößert wird, wenn als Bewegung erfasst wird, dass Daumen und Zeigefinger der Hand einen Kreis bilden, der geöffnet wird, indem sich Daumen und Zeigefinger auseinander bewegen. Weiterhin ist hinterlegt, dass die OP-Leuchte 1 von einem Ruhemodus in einen Bereitschaftsmodus wechselt, wenn zwei Hände in dem Messraum 3 erfasst werden und erfasst wird, dass sich die beiden Hände von außen kommend kreuzen. Entsprechend ist hinterlegt, dass die OP-Leuchte 1 von dem Bereitschaftsmodus in den Ruhemodus wechselt, wenn zwei übereinander gekreuzte Hände auseinandergezogen werden. Ferner ist hinterlegt, dass eine erste Farbtemperatur des von der OP-Leuchte 1 ausgestrahlten Lichts eingestellt wird, wenn der Daumen erfasst wird, eine zweite Farbtemperatur, wenn der Zeigefinger erfasst wird, eine dritte Farbtemperatur, wenn der Mittelfinger erfasst wird und eine vierte Farbtemperatur, wenn der Ringfinger erfasst wird. In anderen Ausführungsbeispielen können weitere Bewegungen zugeordnete Bewegungsdaten weiteren Einstellparametern der OP-Leuchte 1 zugeordnet sein.

In Figur 2 ist die erfindungsgemäße OP-Leuchte 1, die in Figur 1 in einer Seitenansicht dargestellt ist, in einer Prinzipdarstellung von unten dargestellt. In Figur 2 ist der Rahmen 10 der OP-Leuchte 1 erkennbar. Die optische Achse 4 läuft in die Zeichenebene hinein und ist in der Zeichenebene, die senkrecht auf der optischen Achse 4 steht, in der Mitte des Rahmens 10 der OP-Leuchte 1 angeordnet. Entsprechend ist die optische Achse 4 auch in der Mitte des von der OP-Leuchte 1 ausgeleuchteten Bereichs angeordnet. Die optische Achse 4 verläuft durch den Schnittpunkt des in Figur 2 dargestellten Kreuzes.

Der Sensor 2 ist an der Sensorposition 100 am Rahmen 10 der OP-Leuchte 1 angeordnet. In anderen, nicht dargestellten Ausführungsformen, können an weiteren Sensorpositionen 200, 300 und/oder 400 weitere Sensoren 2 der Sensoreinheit der OP-Leuchte 1 angeordnet sein. Insbesondere ist es vorteilhaft, bei einer Anordnung von Sensoren 2 der Sensoreinheit am Rahmen 10 der OP-Leuchte 1 die Sensoren 2 so zueinander anzuordnen, dass stets zwei Sensoren 2 zueinander spiegelsymmetrisch mit der optischen Achse 4 als Spiegelgeraden angeordnet sind. Beispielsweise kann bei einer Anordnung von vier Sensoren 2 an den Sensorpositionen 100, 200, 300 und 400, die somit jeweils einen gleichen Abstand zur optischen Achse 4 aufweisen und jeweils um 90 Grad zueinander versetzt um die optische Achse 4 angeordnet sind, eine besonders gute räumliche Erfassung innerhalb des gesamten Messraums 3 gewährleistet sein. Insbesondere kann in einer vorteilhaften Ausführungsform auch ein Sensor 2 an einer Position entlang der optischen Achse vorgesehen sein, beispielsweise in einem sterilen Griff der OP-Leuchte. Die Anordnung eines Sensors 2 entlang der optischen Achse kann den Vorteil mit sich bringen, dass der Messraum besonders vorteilhaft, insbesondere symmetrisch zur optischen Achse, relativ zu dem ausgeleuchteten Bereich der OP-Leuchte angeordnet ist, was eine besonders komfortable Steuerung der OP-Leuchte ermöglichen kann. Insbesondere kann diese Anordnung eines Sensors 2 eine besonders präzise Positionsbestimmung und Bewegungserkennung des Aktionselements in der Nähe des Operationsfeldes ermöglichen.

Aus den beschriebenen erfindungsgemäßen Ausführungsformen der erfindungsgemäßen OP-Leuchte 1 ist ersichtlich, dass jeweils ein Messraum 3 unterhalb der OP-Leuchte 1 vorgesehen ist, innerhalb dessen Bewegungen eines Aktionselements zur Steuerung der OP-Leuchte 1 erfasst werden können. Die Größe des Messraums 3 kann durch die Ausgestaltung von Sensoren 2 der Sensoreinheit vorbestimmt sein. Insbesondere kann die Größe des Messraums 3 im Hinblick auf bestimmte Anforderungen, die je nach Einsatzgebiet an die OP-Leuchte 1 gestellt werden, wählbar sein.

### Bezugszeichenliste

- 1: OP-Leuchte
- 2: Sensor
- 3: Messraum
- 4: Optische Achse
- 10: Rahmen
- 100: Sensorposition
- 200: Sensorposition
- 300: Sensorposition
- 400: Sensorposition

## Patentansprüche

1. Berührungslos steuerbare OP-Leuchte (1) umfassend eine Steuereinheit und eine Sensoreinheit, wobei die Steuereinheit zum Steuern der OP-Leuchte (1) ausgebildet ist, wobei die Sensoreinheit dazu ausgebildet ist, eine Bewegung eines Aktionselements zu erfassen und in Bewegungsdaten umzuwandeln, die eine Ortsveränderung des Aktionselements während der Bewegung widerspiegeln, wobei die Steuereinheit dazu ausgebildet ist, die von der Sensoreinheit generierten Bewegungsdaten auszulesen und in Abhängigkeit von den Bewegungsdaten die OP-Leuchte (1) zu steuern, wobei die Sensoreinheit so ausgebildet ist, dass sie die Erfassung der Bewegung des Aktionselements in drei Dimensionen in einem Messraum (3) gewährleistet, der in Abstrahlrichtung unterhalb der OP-Leuchte (1) angeordnet ist, wobei die OP-Leuchte (1) einen Ruhemodus aufweist, in dem die Steuereinheit keine Steuerung der OP-Leuchte (1) über die ausgelesenen Bewegungsdaten gewährleistet, sowie einen Bereitschaftsmodus, in dem die Steuereinheit die Steuerung der OP-Leuchte (1) über die ausgelesenen Bewegungsdaten gewährleistet, wobei die Steuereinheit so ausgebildet ist, dass durch eine Initialisierungsaktion der Wechsel von dem Ruhemodus in den Bereitschaftsmodus und durch eine Deaktivierungsaktion der Wechsel von dem Bereitschaftsmodus zu dem Ruhemodus durchführbar ist, **dadurch gekennzeichnet, dass** als Deaktivierungsaktion festgelegt ist, dass während einer vorbestimmbaren Totzeit keine Bewegung des Aktionselements erfasst wurde.

2. OP-Leuchte (1) nach Anspruch 1,
**dadurch gekennzeichnet, dass**
eine optische Achse, entlang der die OP-Leuchte (1) Licht abstrahlt, durch den Messraum (3) verläuft.

3. OP-Leuchte (1) nach Anspruch 2,
**dadurch gekennzeichnet, dass**
der Messraum (3) in einer Ebene senkrecht zur optischen Achse symmetrisch um die optische Achse angeordnet ist.

4. OP-Leuchte (1) nach einem der vorangehenden Ansprüche,
**dadurch gekennzeichnet, dass**
die Sensoreinheit genau einen Sensor (2) umfasst, der an einer Sensorposition (100, 200, 300, 400) an der OP-Leuchte (1) angeordnet ist, wobei der Messraum (3) um die Sensorposition (100, 200, 300, 400) symmetrisch ist.

5. OP-Leuchte (1) nach einem der vorangehenden Ansprüche,
**dadurch gekennzeichnet, dass**
die OP-Leuchte (1) so ausgebildet ist, dass die Steuereinheit die OP-Leuchte (1) nur in Abhängigkeit von solchen Bewegungsdaten steuert, die die Sensoreinheit zu den Bewegungen des Aktionselements generiert, das die Initialisierungsbewegung vollzogen hat.

6. OP-Leuchte (1) nach einem der vorangehenden Ansprüche,
**dadurch gekennzeichnet, dass**
als Initialisierungsaktion eine Initialisierungsbewegung des Aktionselements in dem Messraum (3) festgelegt ist.

7. OP-Leuchte (1) nach einem der vorangehenden Ansprüche,
**dadurch gekennzeichnet, dass**
als Deaktivierungsaktion eine Deaktivierungsbewegung des Aktionselements in dem Messraum festgelegt ist.

8. OP-Leuchte (1) nach einem der vorangehenden Ansprüche,
**dadurch gekennzeichnet, dass**
die Sensoreinheit dazu ausgebildet ist, Bewegungen von nur genau einem Aktionselement zu einer Zeit zu erfassen, wobei die Sensoreinheit die Bewegung des Aktionselements bis zum Abschluss der Bewegung ununterbrochen erfasst und erst nach einer vorbestimmbaren Ruhezeit des Aktionselements die Erfassung eines anderen Aktionselements gewährleistet.

9. OP-Leuchte (1) nach Anspruch 8,
**dadurch gekennzeichnet, dass**
die OP-Leuchte (1) ein Anzeigeelement umfasst, das eine Bereitschaft der OP-Leuchte (1) zur Erfassung des anderen Aktionselements anzeigt.

10. OP-Leuchte (1) nach einem der vorangehenden Ansprüche,
**dadurch gekennzeichnet, dass**
die OP-Leuchte (1) eine Anzeigeeinheit umfasst, die anzeigt, ob die OP-Leuchte (1) durch eine Bewegung des Aktionselements steuerbar ist.

## Claims

1. Surgical lamp (1) controllable without contact, comprising a control unit and a sensor unit, wherein the control unit is configured for controlling the surgical lamp (1), wherein the sensor unit is configured for detecting a movement of an action element and conversion thereof into motion data reflecting a change in the location of the action element during a movement, wherein the control unit is configured for reading the motion data generated by the sensor unit and for controlling the surgical lamp (1) in response to the motion data, wherein the sensor unit is configured in such a manner as to ensure the detection of the movement of the action element in three dimensions in a measuring space (3) arranged in the radiation direction below the surgical lamp (1), wherein the surgical lamp (1) presents a sleep mode in which the control unit does not guarantee control of the surgical lamp (1) via the read-out motion data, and a standby mode in which the control unit guarantees control of the surgical lamp (1) via the read-out motion data, wherein the control unit is configured in a manner such that by an initializing action the change from the sleep mode to the standby mode can be performed and that by a deactivating action the change from the standby mode to the sleep mode can be performed, **characterized in that** a non-detection of any movement of the active element during a dead-time which can be predetermined is set as a deactivation action.

2. Surgical lamp (1) according to claim 1, **characterized in that** an optical axis along which the surgical lamp (1) radiates light runs through the measuring space (3).

3. Surgical lamp (1) according to claim 2, **characterized in that** the measuring space (3) is arranged symmetrically about the optical axis in a plane perpendicular to the optical axis.

4. Surgical lamp (1) according to any one of the preceding claims, **characterized in that** the sensor unit comprises precisely one sensor (2) disposed in a sensor position (100, 200, 300, 400) on the surgical lamp (1), wherein the measuring space (3) is symmetrical about the sensor position (100, 200, 300, 400).

5. Surgical lamp (1) according to any one of the preceding claims, **characterized in that** the surgical lamp (1) is configured in a manner such that the control unit controls the surgical lamp (1) in response to merely those motion data the sensor unit generates regarding the movements of the action element that has completed the initializing movement.

6. Surgical lamp (1) according to any one of the preceding claims, **characterized in that** as an initializing action there is fixed an initializing movement of the action element in the measuring space (3).

7. Surgical lamp (1) according to any one of the preceding claims, **characterized in that** as a deactivating action there is fixed a deactivating movement of the action element in the measuring space (3).

8. Surgical lamp (1) according to any one of the preceding claims, **characterized in that** the sensor unit is configured for detecting movements of precisely one action element at a time, wherein the sensor unit detects the movement of the action element continuously until the movement is completed and guarantees the detection of another action element only after a pre-determinable idle time of the action element.

9. Surgical lamp (1) according to claim 8, **characterized in that** the surgical lamp (1) comprises an indicator element indicating readiness of the surgical lamp (1) for the detection of the other action element.

10. Surgical lamp (1) according to one of the preceding claims, **characterized in that** the surgical lamp (1) comprises an indicator unit indicating whether the surgical lamp (1) is controllable by a movement of the action element.

## Revendications

1. Lampe de bloc opératoire à commande sans contact (1), comprenant une unité de commande et une unité de détection, dans laquelle l'unité de commande est configurée pour commander la lampe de bloc opératoire (1), l'unité de détection est configurée pour détecter un mouvement d'un élément d'action et pour convertir ce mouvement en des données de mouvement réfléchissant un changement de l'emplacement de l'élément d'action pendant le mouvement, l'unité de commande étant configurée pour lire les données de mouvement générées par l'unité de détection et pour commander la lampe de bloc opératoire (1) en fonction des données de mouvement, l'unité de détecteur étant configurée de sorte qu'elle garantisse la détection du mouvement de l'élément d'action en trois dimensions dans un espace de mesure (3) disposé au-dessus de la lampe de bloc opératoire (1) dans la direction du rayonnement, la lampe de bloc opératoire (1) présentant un mode inopérant dans lequel l'unité de commande ne garantit aucune commande de la lampe de bloc opératoire (1) par les données de mouvement lues ainsi qu'un mode d'attente dans lequel l'unité de commande garantit la commande de la lampe de bloc opératoire (1) par les données de mouvement lues, l'unité de commande étant configurée de telle manière que, par une action d'initialisation, le mode inopérant peut passer au mode d'attente et, par une action de désactivation, le mode d'attente peut passer au mode inopérant, **caractérisée** en ce la détection qu'aucun mouvement de l'élément d'action n'a été détecté pendant un temps mort pouvant être prédéterminé est définie comme une action de désactivation.

2. Lampe de bloc opératoire (1) selon la revendication 1, **caractérisée en ce qu'**un axe optique le long duquel la lampe de bloc opératoire (1) émet de la lumière, passe par l'espace de mesure (3).

3. Lampe de bloc opératoire (1) selon la revendication 2, **caractérisée en ce que** l'espace de mesure (3) est arrangé de manière symétrique autour l'axe optique dans un plan perpendiculaire à l'axe optique.

4. Lampe de bloc opératoire (1) selon l'une des revendications précédentes, **caractérisée en ce que** l'unité de détection comporte exactement un détecteur (2) disposé sur la lampe de bloc opératoire (1) dans une position de détection (100, 200, 300, 400), l'espace de mesure (3) étant symétrique autour la position (100, 200, 300, 400) du détecteur.

5. Lampe de bloc opératoire (1) selon l'une des revendications précédentes, **caractérisée en ce que** la lampe de bloc opératoire (1) est configurée de sorte que l'unité de commande seulement commande la lampe de bloc opératoire (1) en fonction de telles données de mouvement qui sont générées par l'unité de détection aux mouvements de l'élément d'action qui a complété le mouvement d'initialisation.

6. Lampe de bloc opératoire (1) selon l'une des revendications précédentes, **caractérisée en ce que** comme action d'initialisation on a déterminé un mouvement d'initialisation de l'élément d'action dans l'espace de mesure (3).

7. Lampe de bloc opératoire (1) selon l'une des revendications précédentes, **caractérisée en ce que** comme action de désactivation on a déterminé un mouvement de désactivation de l'élément d'action dans l'espace de mesure.

8. Lampe de bloc opératoire (1) selon l'une des revendications précédentes, **caractérisée en ce que** l'unité de détection est configurée pour détecter les mouvements d'exactement un seul élément d'action à la fois, l'unité de commande détectant le mouvement de l'élément d'action jusqu'à la fin du mouvement sans interruption, la détection d'un autre élément d'action étant garantie seulement après une période de repos prédéterminable de l'élément d'action.

9. Lampe de bloc opératoire (1) selon la revendication 8, **caractérisée en ce que** la lampe de bloc opératoire (1) comporte un élément indicateur indiquant la disponibilité de la lampe de bloc opératoire (1) pour la détection de l'autre élément d'action.

10. Lampe de bloc opératoire (1) selon l'une des revendications précédentes, **caractérisée en ce que** la lampe de bloc opératoire (1) comporte un élément indicateur indiquant si la lampe de bloc opératoire (1) peut être commandée par un mouvement de l'élément d'action.
